(19) Europäisches Patentamt

European Patent Office

Office européen des brevets

(11) Publication number: **0 188 413**
**B1**

(12) EUROPEAN PATENT SPECIFICATION

(45) Date of publication of patent specification: **26.09.90**

(21) Application number: **84902885.7**

(22) Date of filing: **16.07.84**

(86) International application number:
**PCT/US84/01117**

(87) International publication number:
**WO 86/00539 30.01.86 Gazette 86/03**

(51) Int. Cl.⁵: **A 61 N 1/32**

(54) CIRCUIT APPARATUS FOR ELECTROTHERMAL TREATMENT OF CANCER EYE.

(43) Date of publication of application:
**30.07.86 Bulletin 86/31**

(45) Publication of the grant of the patent:
**26.09.90 Bulletin 90/39**

(84) Designated Contracting States:
**BE DE FR SE**

(56) References cited:
**US-A-3 812 858**
**US-A-4 016 886**
**US-A-4 074 719**
**US-A-4 124 030**
**US-A-4 189 685**
**US-A-4 237 898**

(73) Proprietor: **RDM INTERNATIONAL, INC.**
**1 West Deer Valley Road Suite 300**
**Phoenix, Arizona 85027 (US)**

(72) Inventor: **MIODUSKI, Paul**
**3470 M. Olfen**
**Tucson, AZ 85719 (US)**

(74) Representative: **Wächtershäuser, Günter, Dr.**
**Tal 29**
**D-8000 München 2 (DE)**

Courier Press, Leamington Spa, England.

**Description**

The present invention relates to an apparatus for electrothermal treatment of unhealthy tissue in accordance with the prior art portion of claim 1.

More specifically, the present invention relates to an apparatus for treating malignant tissue known as "cancer eye" in the eyes of livestock by applying high frequency current, by means of spaced probes, to the malignant tissue to increase the temperature thereof to a level which is high enough to kill the malignant tissue but is low enough to avoid permanent damage to the adjacent healthy tissue.

Benign and malignant tumors of the eye and eyelid in cattle are generally referred to by the term "cancer eye". Approximately 80% of such tumors are malignant and many of the rest become malignant with time. Cancer eye is a serious problem throughout the United States, especially in high elevation locations where solar radiation is most intense. As pointed out by his article "Electrothermal Treatment of Cancer Eye" by James D. Doss, published in the August 1977 issue of the LASL Mini-Review, 77-14, published in 1975 by the Los Alamos Scientific Laboratory of the University of California at Los Alamos, New Mexico, cancer eye was the leading individual cause of cattle carcass condemnation at slaughter houses inspected by the United States Department of Agriculture. In that year, losses due to cancer eye were thought to exceed $20,000,000.00 per year in the United States alone. As a result of research at the above-mentioned Los Alamos Scientific Laboratory, techniques have been developed for on-range use involving passage of high frequency current through malignant tissue to increase its temperature to approximately 50°C. (122°F.) for thirty seconds, resulting in effective arresting of early discovered cases of cancer eye in cattle. Such temperature preferentially kills cancer cells, which are usually more susceptible to permanent damage by heat than healthy cells.

US-A-4 189 685 discloses an apparatus for electrothermal treatment of cancerous or tumorous tissue in accordance with the above-mentioned type, including a circuit for producing flow of a high frequency current through the tissue to heat it without damaging adjacent healthy tissue, said electrothermal apparatus including first and second spaced current probes each having a contact surface for electrically contacting the surface of said tissue to thereby conduct said high frequency electrical current through said tissue, said electrothermal treatment apparatus also including oscillating circuit means for producing a high frequency voltage signal for application across said first and second current probes to cause said high frequency current to flow through said tissue if said contact surfaces are held sufficiently forcefully against the surface of said tissue, said circuit comprising in combination: temperature sensing means for sensing the temperature of said first current probe to produce a first electrical signal representative of the temperature of said first probe; circuit means responsive to said first electrical signal for producing a second electrical signal and audio transducer means responsive to said second electrical signal for producing an audible sound. The temperature sensing means formed by a thermo-element causes a switching circuit to feed the high frequency current to the probes until the thermo-element detects a temperature level corresponding to the coagulation-causing temperature. A control circuit is arranged so as to keep the temperature essentially steady when this temperature level is reached. However, there may occur the problem that the initial surge of current actually heats up the tissue so rapidly that the temperature increase of the sensor located in one of the probe tips lags the tissue temperature so that the circuitry cannot adequately regulate the amount of high frequency current applied to the unhealthy tissue before overheating of the tissue occurs. Such overheating can permanently damage healthy tissue which, of course, is highly undesirable.

In view of this prior art, the present invention is based on the object of providing an apparatus for electrothermal treatment of unhealthy tissue of the above-mentioned type preventing an overheating of the tissue.

This object is achieved by an apparatus in accordance with the prior art portion of claim 1 having the features indicated in the characterizing portion thereof.

It is an advantage of the apparatus in accordance with the present invention that excessive initial temperature overshoot and dissipation and waste of power is prevented. In other words, the temperature of the cancer eye tissue is accurately maintained within a predetermined range during the treatment procedure. An excessively rapid initial build-up of temperature in the cancer eye tissue is automatically avoided.

Hereinafter, a preferred embodiment of the apparatus for electrothermal treatment in accordance with the present invention will be described with reference to the attached drawings, in which:

Fig. 1 is a circuit schematic diagram of the circuitry of the invention.

Fig. 2 is a circuit schematic diagram of a ring oscillator used in the circuit of Fig. 1.

Fig. 3 is a perspective view of the electrothermal treatment apparatus in which the circuit of Fig. 1 is utilized.

Fig. 4 is a diagram of several waveforms that are useful in illustrating the operation of the circuit of Fig. 1.

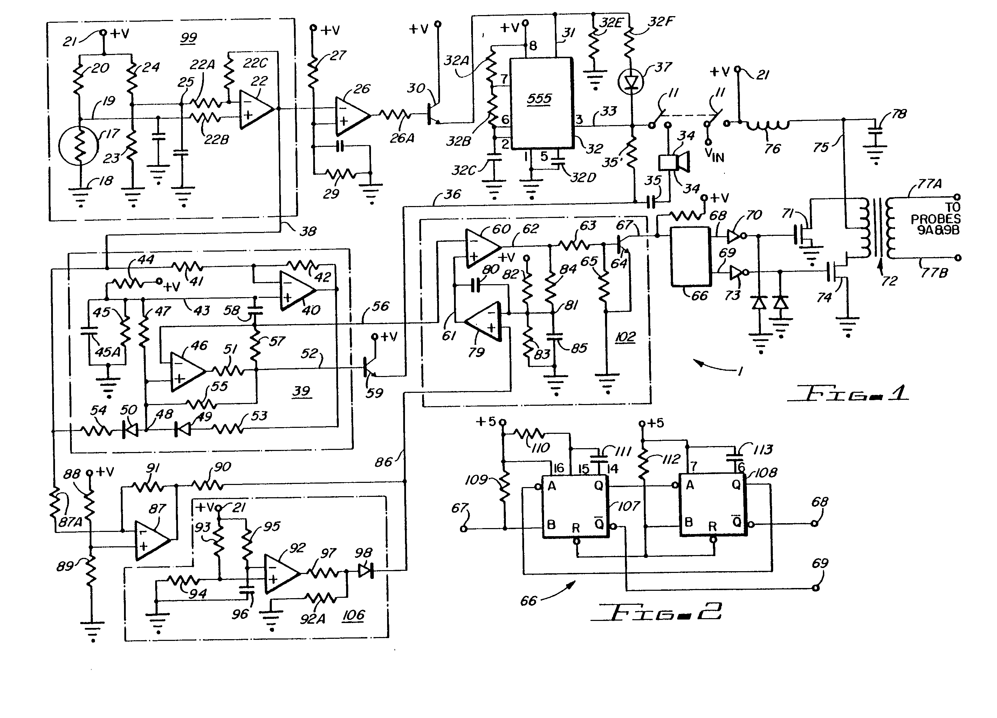

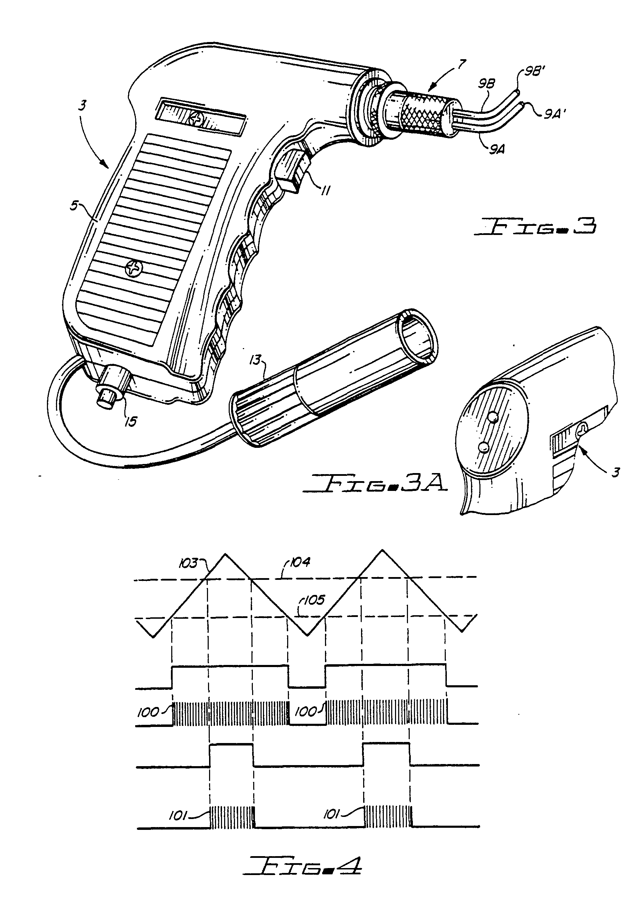

Referring now to the drawings, the circuit of Fig. 1 is positioned in the handle 5 of the electrothermal treatment apparatus 3 shown in Fig. 3. Apparatus 3 includes a momentary switch 11 which can be actuated by the index finger of a person gripping handle 5 to actuate circuit 1. When circuit 1 is actuated, a high frequency (approximately 2 Mhz) output voltage produced thereby appears across two spaced, electrically conductive electrically isolated probes 9A and 9B. If probes 9A and 9B are held against cancerous tissue in the eye of a livestock animal with adequate pressure, the resulting contact resistances will be sufficiently low that voltage between probes 9A and 9B causes a high frequency current to flow from one of the probes through the cancerous tissue into the other. This causes resistive heating of the cancerous tissue. As previously explained, the technique of treating "cancer eye" in cattle has been proven to be quite effective.

Reference numeral 15 of Fig. 3 designates a main switch that makes power supplied via an electrical connector 13 available to circuit 1 on conductor 21.

Referring now to Fig. 1, the structure of circuit 1 will be set forth in detail. Thermistor 17, which is located in the tip of one of probes 9A and 9B, is connected between ground conductor 18 and conductor 19. The purpose of thermistor 17 is to determine the temperature to which the cancerous tissue has been raised. Thermistor 17 is connected in series with resistor 20, the upward end of which is connected to a supply voltage conductor 21 that has a potential of +V volts. Actuation of switch 11 applies the voltage $V_{in}$ to conductor 21 to actuate circuit 1. ($V_{in}$ is made available by means of switch 15 of Fig. 3).

The voltage on conductor 19 is applied by means of a resistor 22B to the positive input of operational amplifier 22. Resistors 23 and 24 are connected in series between +V and ground to produce a reference voltage on conductor 25. The voltage on conductor 25 is applied by a resistor 22A to the negative input of operational amplifier 22, so that when the voltage on conductor 19 exceeds the reference voltage on conductor 25, the output of operational amplifier 22 goes to a high level. (Note that operational amplifiers 22, 26, 40 and 46 of Fig. 1 can be implemented by means of an LM324N integrated circuit quad operational amplifier.)

The output of operational amplifier 22 is connected to the negative input of operational amplifier 26. The positive input of operational amplifier 26 is connected to the junction between resistors 27 and 29, which are connected in series between +V and ground to establish a switching point for operational amplifier 26 that corresponds to a thermistor temperature of 50°C.

The output of operational amplifier 26 is coupled to the base of NPN transistor 30, the collector of which is connected to +V, its emitter being connected to conductor 31. Conductor 31 is connected to the reset input of a "555" integrated circuit timer, which is widely available. The 555 timer is designated by reference numeral 32, and is connected as shown to provide a two second oscillation frequency at its output on conductor 33. When momentary switch 11 is held closed, the output of timer 32 is connected to one input of a ceramic transducer 34, which functions as an audio frequency speaker. Conductor 33 serves as a "ground return" line for transducer 34, the other terminal of which is coupled by capacitor 35 to conductor 36, to which an audio frequency signal proportional in magnitude to the temperature of thermistor 17 is applied, as subsequently explained.

Light emitting diode 37 is also connected to conductor 33, and blinks in synchronization with oscillation of timer 32 at a two second repetition rate.

The output of operational amplifier 22 produces an amplified voltage on conductor 38 proportional to the temperature of thermistor 17. This voltage is applied to the input of a voltage controlled oscillator (VCO) circuit 39. VCO circuit 39 includes operational amplifier 40, the negative input of which is connected by resistor 41 to conductor 38. Resistor 42 connects the output of operational amplifier 40 back to the negative input thereof.

The positive input of operational amplifier 40 is connected to conductor 43 which, in turn, is connected to the junction between resistors 44 and 45. Resistors 44 and 45 are connected in series between +V and ground. The positive of input of operational amplifier 46 is connected by resistor 47 to conductor 43 and is also connected to the junction 48 between the cathode of diode 49 and the anode of diode 50. The output of operational amplifier 46 is connected by resistor 51 to conductor 52, which is one of the outputs of VCO circuit 39. The anode of diode 49 is connected by resistor 53 to the output of operational amplifier 40. The cathode of diode 50 is connected by resistor 54 to conductor 38. Conductor 48 is connected by resistor 55 to conductor 52.

The negative input of operational amplifier 46 is connected to conductor 56, which is a second output of VCO circuit 39. Conductor 56 is connected by resistor 57 to conductor 52 and also is connected by capacitor 58 to conductor 43.

Those skilled in the art will realize the VCO circuit 39 produces a triangular waveform signal on conductor 56 and a square wave signal on conductor 52, and that the frequency on both such waveforms is proportional to the voltage on conductor 38 and hence to the temperature of thermistor 17.

The square wave signal on conductor 52 is applied to the base of NPN transistor 59, the collector of which is connected to +V and the emitter of which is connected to conductor 36 to thereby apply an audio frequency signal to one input of audio transducer 34. Thus, it is seen that the pitch of the sound emitted by audio transducer 34 is proportional to the temperature of transducer 17.

The triangular waveshape produced on conductor 56 is applied to the negative input of operational amplifier 60, which functions as a comparator in this case. (Note that operational amplifiers 60, 79, 87 and 92 also can be implemented by means of LM324N integrated circuit quad op amps. Note also that the op amps can be connected to function as comparators.) The triangular waveform on conductor 56 is compared with the DC voltage on conductor 61, which is connected to the positive input of comparator 60 to establish the switching point of comparator 60. The output of comparator 60 is connected to conductor 62, which is coupled by resistor 63 to the base of NPN transistor 64. The base of transistor 64 is connected by resistor 65 to ground. The emitter of transistor 64 is connected to ground, and the collector is connected to a duty cycle control input of a ring oscillator circuit 66.

As subsequently explained with reference to Fig. 2, oscillator 66 is set to oscillate at approximately 2 megahertz. It has a duty cycle control input connected to conductor 67 which halts the oscillation when that input is at a logical "0". Conductor 67 is connected to the collector of transistor 64. The signal produced on conductor 67 in effect modulates the "duty cycle" of the two megahertz bursts produced on output conductors 68 and 69 of ring oscillator circuit 66. Conductor 68 is connected to the input of an inverter-driver circuit 70, the output of which is connected to the gate electrode of a VMOS power field effect transistor 71. The source electrode of VMOS transistor 71 is connected to ground, and its drain electrode is connected to one primary terminal of transformer 72. Conductor 69 is connected to the input of inverter-driver 73, the output of which is connected to the gate electrode of VMOS power transistor 74. The source electrode of transistor 74 is connected to ground and its drain electrode is connected to the other primary winding terminal of transformer 72.

A center tap electrode 75 of the primary winding of transformer 72 is coupled by inductor 76 to +V conductor 21. Capacitor 78 is connected between center tap 75 and ground. The terminals of the secondary winding of transformer 72 are connected by conductors 77A and 77B to probes 9A and 9B, respectively, of electrothermal apparatus 3 of Fig. 3.

Circuitry 102 performs the function of regulating the duty cycle control signal applied to oscillator 66 to maintain the temperature of the cancerous or tumerous tissue in the range between 50°C. and 55°C. The threshold level applied by conductor 61 to the positive input of comparator 60 normally represents the temperature of thermistor 17 in the range from 50°C. to 55°C., so that the "duty cycle" of the two megahertz voltage applied to probes 9A and 9B is automatically varied to keep it in the range between 50°C. and 55°C. To accomplish this, operational amplifier 79 has its output connected by conductor 61 to the negative input of comparator 60. The negative input of comparator 79 is connected by means of capacitor 80 to conductor 61. The negative input of comparator 79 also is connected to conductor 81, which is connected to the junction between resistors 82 and 83. Resistors 82 and 83 are connected in series between −V and ground. Conductor 81 is also connected to the junction between resistor 84 and capacitor 85, the other terminal of resistor 84 being connected to conductor 62 and the other terminal of capacitor 85 being connected to ground.

The positive input of operational amplifier 79 is connected to conductor 86.

Conductor 38, previously referred to, is connected by resistor 87A to the input of operational amplifier 87. The positive input of operational amplifier 87 is connected to the junction between resistors 88 and 89, which are connected in series between +V and ground. The output of operational amplifier 87 is connected by resistor 90 to conductor 86 and by resistor 91 to the negative input thereof.

The immediately foregoing circuitry performs a "scaling" function on the voltage produced in response to thermistor 17 by operational amplifier 22 in order to produce a scaled voltage representative of the temperature of thermistor 17 in the range from 50 to 55°C. on conductor 86.

Circuitry 106 performs the function of causing circuitry 102 to impose a reduced "duty cycle" of approximately 50% on the duty cycle control signal applied to oscillator 66 for the first 8 seconds that power is applied to circuit 1. Reference numeral 92 designates a comparator having its positive input connected to the junction between resistors 93 and 94, which are connected in series between +V and ground to establish a reference voltage equal to approximately two-thirds of the value of +V. The negative input of operational comparator 92 is connected to the junction between resistor 95 and capacitor 96, which are connected in series between +V and ground to produce a slowly rising signal when +V volts is applied to conductor 21 in response to closing of switch 11. The output of comparator 92 is coupled by resistor 97 to the anode of diode 98, the cathode of which is connected to conductor 86. The immediately foregoing circuit cooperates with circuitry 102 to limit the "duty cycle" of oscillator 66 to approximately 50% for the first eight seconds after switch 11 is actuated. For the first eight seconds, comparator 92 produces a high output voltage by forward biasing diode 98 and establishes a switchpoint at the positive input of comparator 79. This switchpoint voltage is determined by the value of resistors 97 and 92A and by the forward voltage drop of diode 98.

The operation of circuit 1 will now be explained with reference to the waveforms of Fig. 4.

First, after immobilizing the animal's eye by means of a spoon which is inserted behind the eyeball, the user holds the electrothermal treatment apparatus 3 of Fig. 3 in his hand and presses the lower probe contact surfaces 9A' and 9B' (Fig. 3) against the cancerous tissue and then depresses momentary switch 11. This closes the two switch "wiper" elements designated by reference numeral 11 in Fig. 1 and applies +V volts to conductor 21 of circuit 1. (It is assumed that switch 15 has been closed, making the voltage $V_{in}$ available to circuit 1). It should be borne in mind that the animal may be struggling and that the user may experience considerable difficulty in maintaining contact surfaces 9A' and 9B' against the cancer eye tissue with a sufficient amount of pressure to ensure adequate electrical contact. Obviously, if inadequate contact of areas 9A' and 9B' against the cancerous tissue is maintained, this will increase the impedance load on the output of circuit 1. The increased impedance will decrease the amount of two megahertz current flowing through probes 9A and 9B and the cancerous tissue, preventing its temperature from being raised to an adequate level.

With this in mind, it will be recognized that if adequate probe contact pressure is maintained against the cancerous tissue, the temperature of the tissue will begin to rise quite rapidly. As the temperature of the tissue rises, the temperatures of probes 9A and 9B also rise, although with a slight lag in time which may vary from a fraction of a second to more than one second. The temperature of thermistor 17 is almost exactly equal to the temperature of the one of probes 9A and 9B in which it is disposed. The circuit within block 99 amplifies the voltage across thermistor 17, producing a voltage on conductor 38 which is proportional to the thermistor temperature. When the thermistor temperature reaches approximately 50°C., the voltage on conductor 38 exceeds the threshold voltage applied to the positive input of comparator 26. Comparator 26 then switches, causing transistor 30 to apply an enable input to timer 32, enabling it to function as an astable multi-vibrator with a two second period on its output 33.

Meanwhile, as soon as the temperature of thermistor 17 begins to increase, the voltage on conductor 38 also begins to increase, and VCO circuit 39 begins to oscillate at an audio frequency that increases at a rate that is proportional to the increase in the voltage on conductor 38. VCO circuit 39 produces a square wave signal on conductor 36 which is applied via capacitor 35 to one terminal of audio transducer 34. Until timer 32 begins switching at its two second repetition rate, the voltage on conductor 33 is at +V. (Switch 11, of course, is closed.) The square wave produced on conductor 36 is smoothed somewhat by capacitor 35 and resistor 35', producing a relatively pure sinusoidal waveform of gradually increasing pitch across transducer 34, causing an audible sound of increasing pitch to be emitted by transducer 34. The range of audio frequencies of the sound is roughly 1 kilohertz to 4 kilohertz.

The user of electrothermal apparatus 3 knows that as long as the pitch of the sound emitted by transducer 34 continues to increase at a proper rate, he is maintaining adequate pressure of contact surfaces 9A' and 9B' against the cancerous tissue because its temperature is increasing at the same rate as the pitch of the sound emitted by transducer 34. If adequate contact is momentarily lost due, for example, to the struggling of the animal, the user realizes this immediately, ' because the pitch of the sound emitted by transducer 34 immediately stops increasing, and may, in fact, begin decreasing if the temperature of thermistor 17 begins to fall sharply due to loss of adequate probe surface contact with the cancerous tissue.

When adequate pressure of the probe contact surface has been maintained with the cancerous tissue for a sufficiently long period of time (typically approximately 10 seconds) the temperature of thermistor 17 will reach 50°C., and the voltage on conductor 33 produced by timer 32 will begin to rise and fall with a two second repetition rate, thereby periodically interrupting the ground return connection of ceramic transducer 34, so that the sound emitted thereby beeps at a two second repetition rate. As long as the temperature of the tissue is at least 50°C., the beeping will continue and the user can count the number of beeps until 15 of them have occurred, indicating that the cancerous tissue has been held at least 50°C. for at least 30 seconds. At this point, the probes can be removed and switch 11 can be released.

The circuit of Fig. 1 automatically regulates the temperature of the cancerous tissue, i.e., of thermistor 17, by modulating the "duty cycle" of the two megahertz current conducted via probes 9A and 9B to the cancerous tissue. To understand how this is done, it must be realized that ring oscillator 66 produces two non-overlapping square wave signals on conductor 68 and 69. These square wave signals are buffered by driver circuits 70 and 73, respectively, which alternately turn VMOS power transistors 71 and 74 on and off in such a manner that at no time are both transistor 71 and 74 on simultaneously. (Note that VMOS power transistors 71 and 74 can be the IRF523 or higher voltage IRF522 VMOS transistors manufactured by International Rectifier Corporation). The repetition rate of the switching of each of power transistors 71 and 74 is approximately two megahertz. After the temperature of thermistor 17 rises to approximately 50°C., the modulation signal produced on conductor 67 varies the "duty cycle" of the two megahertz "burst" applied to the primary winding of transformer 72 to keep the temperature of thermistor 17 in the range from 50°C. to 55°C. In Fig. 4, waveform 100 illustrates the bursts of two megahertz current applied through probes 9A and 9B at a relatively high "duty cycle" corresponding to a thermistor temperature at the low end of the range from 50°C. to 55°C., whereas waveform 101 represents a relatively low duty cycle of the bursts of two megahertz current that would be produced by the automatic duty cycle regulating circuit represented by reference numeral 102 in Fig. 1 for a thermistor temperature at the high end of the range from 50°C. to 55°C. It is to be understood that the term "duty cycle" as used herein refers to the duty cycle of the "envelope" of the bursts of 2 MHz current, and not to each individual cycle of the 2 MHz current. However, the true duty cycle of each cycle of the 2 MHz current also could be varied to regulate the power delivered to the cancerous tissue.

In Fig. 4, reference numeral 103 represents the above-mentioned triangular waveform produced by VCO circuit 39 on conductor 56. Dotted line 104 represents a relatively high threshold or trip point of comparator 79, applied as a voltage on conductor 86 for a relatively high thermistor temperature near 55°C. When the voltage on conductor 56 is above the voltage represented by dotted line 104 the output of comparator 60 produces a signal that drives transistor 64 and starts two megahertz oscillator circuit 66, resulting in the two megahertz burst of pulses represented by reference numeral 101; this burst lasts as long as the triangular wave shape 103 is above threshold 104. As an alternate example, if the thermistor temperature is at the low end of the range from 50°C. to 55°C., a higher "duty cycle" of the current bursts is needed to pump more two megahertz current into the cancerous tissue, and a lower threshold voltage will appear on conductor 86. This lower threshold voltage is represented by dotted line 105 in Fig. 4, and comparator 60 functions as described above to produce wider bursts of two megahertz current, such as the ones indicated by reference numeral 100 in Fig. 4.

As previously mentioned, under certain circumstances, the thermal lag in the temperature of the probe in which thermistor 17 is disposed relative to the rapidly rising temperature of the cancerous tissue during the initial period immediately after switch 11 is closed can result in temperature "overshoot" of the cancerous tissue to the extent that healthy eye tissue may be damaged. In order to prevent this occurrence, the invention provides the abovementioned automatic initial eight second period in which the duty cycle of the two megahertz current is limited to 50% for the first eight seconds of operation. This ensures that the temperature of thermistor 17 rises nearly as fast as the temperature of the cancerous tissue, thereby ensuring that the temperature sensed by thermistor 17 accurately represents the temperature of the cancerous tissue. The circuitry designated by reference numeral 106 in Fig. 1 operates to produce a slowly rising waveform that is applied to the negative input of comparator 92, beginning at the instant that switch 11 is closed. Current through resistor 95 charges up capacitor 96 to produce this signal which, when it equals a voltage of approximately two thirds of +V (which typically is approximately 8 volts), causes the output of comparator 92 to fall and go to zero volts. This reverse biases diode 98, allowing the voltage on conductor 86 to be determined by operational amplifier 87, which scales the temperature sensitive voltage produced by thermistor sensing circuit 99. Therefore, after the eight second delay time has elapsed, circuit 1 functions as previously described. However, during that initial eight second time period, the output of comparator 92 is high, diode 98 is forward biased, thereby maintaining the positive input of comparator 79 in circuitry 102 at the above-mentioned relatively high level.

The operation of the duty cycle regulating circuit generally designated by reference numeral 102 in Fig. 1 is as follows. First, comparator 60 compares the triangular waveform 103 (Fig. 4) with the output of operational amplifier 79, which is produced on conductor 61. The positive input of operational amplifier 79 is connected to conductor 86. (Recall that the voltage on conductor 86 is equal to the voltage at the junction between resistors 97 and 92A in circuit 106 for approximately the first eight seconds of operation to force the duty cycle to be approximately 50%. After the first eight seconds of operation have elapsed, the voltage on conductor 86 is a scaled down voltage that is proportional to the temperature of thermistor 17.)

In either case, the voltage on conductor 86 is compared with the voltage on conductor 81 in duty cycle regulating circuit 102, since the latter voltage is applied to the negative input of operational amplifier 79, as operational 79 actually functions as a comparator in this circuit.

The circuitry including resistor 84, capacitor 85, operational amplifier 79 and capacitor 80 functions as an integrator which averages the voltage signal on conductor 62. If the temperature of thermistor 17 is above 50°C., or during the first eight seconds of operation, the voltage on conductor 62 will be a sequence of pulses, the duty cycle of which depends on the voltage on conductor 86. After the first eight seconds of operation, but before the temperature of thermistor 17 attains 50°C., the voltage on conductor 62 will be a logical "1", rather than a sequence of pulses. During this time, the voltage on conductor 81 is equal to the voltage on conductor 62. Operational amplifier 79 compares the voltage on conductor 81 with the voltage conductor on 86, and forces the voltage on conductor 61 to be +V. That causes comparator 60 to force the voltage on conductor 62 to equal the +V (i.e., to have a 100% duty cycle). However, once the duty cycle of the voltage produced on conductor 62 becomes less than 100%, (i.e., after the temperature thermistor 17 exceeds 50°C.) then the above-mentioned integrating circuit averages that value. Then the operation of the duty cycle regulating circuit 102 is as follows. Operational amplifier 79 compares the average of the duty cycle voltage on conductor 62 with the voltage on conductor 86. Then operational amplifier 79 sets the threshold on conductor 61 such that comparator 60 forces the average voltage on conductor 62 to be equal to the present reference voltage on conductor 86. For the component values indicated in Appendix I, this operation results in linear reduction of the duty cycle of the signal on conductor 62, and hence, on conductor 67, with respect to increasing temperature of thermistor 17 from approximately 50°C to approximately 55°C.

The details of ring oscillator circuit 66 can be described with reference to Fig. 2, wherein it can be seen that the major components of ring oscillator 66 include two one-shot integrated circuits 107 and 108 (each of which can be implemented by means of 74123N integrated circuits.) They can be interconnected in the manner shown with resistors 109, 110, and 112 and capacitors 111 and 113 selected to produce a two megahertz oscillation frequency. This circuit is well known to those skilled in the art, and its operation need not be described, except to note that it produces two out-of-phase, two megahertz square wave signals on conductors 68 and 69 whenever conductor 67 is held high.

An important advantage of the circuit described in Fig. 1 relates to the use of high power VMOS transistors 71 and 74 to drive the primary winding of transformer 72. In the past, oscillator circuits used in electrothermal apparatus for treatment of eye cancer have utilized a NPN bipolar transistor in a Collpitts oscillator or a Hartley oscillator to generate the two megahertz frequency. The bipolar power transistor of such circuits is always at least slightly on, and therefore continuously conducts current. The highest magnitude of current occurs when the transistor is saturated, and a very large amount of power is dissipated as a result of this current flowing across the $V_{sat}$ voltage drop between the collector and the emitter of the bipolar NPN transistor. This has resulted in excessive waste of power. It is highly desirable that the electrothermal treatment apparatus used for treating eye cancer in cattle be both portable and battery powered. The excess waste of power of the prior device reduces the number of treatments that can be performed before the batteries need to be re-charged; the excessive power dissipation has also resulted in the handle of some prior devices becoming excessively hot.

The device of Fig. 3, utilizing the circuit of Fig. 1 therein, completely avoids these problems, and is capable of delivering over 90% of the power supplied from the battery through the probes 9A and 9B to the cancerous tissue, if VMOS transistors having sufficiently low channel resistance are used. This occurs because the channel resistance of VMOS devices 71 and 74 is very low, less than a fraction of an ohm, and MOS transistors inherently have no $V_{sat}$ voltage across which the excessive power dissipation of prior devices occurs. The use of essentially square wave current waveforms, which is what are produced by VMOS transistors 71 and 74 and transformer 72, results in high harmonic frequency content of the current passing through the tissue. It is thought, although it has not yet been proved, that this may result in improved heating of the tissue.

Since the pitch of the audible sound represents the temperature of the tissue, the rate of increase of the pitch can also indicate if the rf power being supplied to the probes is adequate to heat the tissue being contacted by the probe. An improved embodiment of the invention is contemplated wherein the power output level of the $V_{MOS}$ transistors 71 and 74 and transformer 72 is adjustable by the user. This would be important in instances wherein the electrothermal device would be used for treatment of a wide range of sizes of tumerous tissue. More power would obviously be required for larger tumors. If too low a power setting were used for a large tumor, failure of the pitch of the audible sound to increase at a predetermined rate despite adequate probe pressure being maintained would alert the user that the power level should be increased. Although the described embodiment of the invention requires the user to count the number of audible beeps to ensure that the tumerous tissue has been maintained at an adequately long time initially, it would be quite feasible to add timing circuitry to count and display the number of seconds that the tissue has been held above a predetermined temperature such as 50°C. Although the described embodiment of the invention provides a 50% duty cycle for the first eight seconds to avoid temperature overshoots, it would be quite feasible to use some other approach, such as gradually increasing the duty cycle from an initial low level to high level. Although various government regulations must be complied with for apparatus and methods used for human medical treatment, it is believed that the described embodiment of the invention can be modified to effectively treat certain skin growths, such as warts, in humans.

## Claims

1. Apparatus for electrothermal treatment of unhealthy tissue including a circuit (1) for producing a flow of a high frequency signal through the unhealthy tissue,

said electrothermal apparatus including first and second spaced current probes (9A, 9B) each having a contact surface for electrically contacting the surface of said unhealthy tissue,

said circuit including output oscillating circuit means (66) for producing a high frequency signal for application across said first and second current probes (9A, 9B),

said output oscillating circuit means including a control input (67),

said circuit comprising:

(a) temperature sensing means (17) for sensing the temperature of said first current probe to produce a first electrical signal (38) representative of the temperature of said first probe; characterized in that said circuit further comprises:

(b) delay circuit means (106) for reducing the duty cycle of said high frequency signal to a predetermined level for a predetermined initial time period after initial application of operating power to said circuit;

(c) means (60, 79) determining said control input (67) for halting oscillation of said output oscillating circuit means (66) when said control input (67) is at a first signal level; and

(d) regulating circuit means (102) responsive to said first electrical signal (38) and coupled to so said control input (67) for periodically interrupting oscillating of said output oscillating circuit means after said predetermined initial time period in order to reduce a duty cycle of said high frequency signal.

2. Apparatus as claimed in claim 1, characterized in that said output oscillating circuit means comprises:

(e) voltage controlled oscillating circuit means (39) responsive to said first electrical signal (38) for producing a second electrical signal (36) and varying the frequency of said second electrical signal in accordance with variations in the temperature of said first current probe (9A), said second electrical signal having a frequency that varies between first and second predetermined audio frequencies as the temperature of said first current probe varies between first and second predetermined temperature levels; and,

that said circuit (1) further comprises:

(f) audio transducer means (34) responsive to said second electrical signal for producing an audible sound, the pitch of which represents the temperature of said tissue when said tissue is being heated by said high frequency signal.

3. Apparatus as claimed in claim 1 or 2, characterized in that said regulating circuit means (102) is responsive to said delay circuit means (106) for causing the duty cycle of said duty cycle signal to have said predetermined duty cycle during said predetermined time period regardless of the temperature of said first current probe (9A).

4. Apparatus as claimed in one of the claims 1 to 3, characterized by threshold circuit means (26) responsive to said first electrical signal for producing a third electrical signal if said first electrical signal exceeds a predetermined level and

timing circuit means (32) responsive to said third electrical signal and coupled to said audio transducer means for effecting interruption of said audio sound at a predetermined repetition rate.

5. Apparatus as claimed in one of the claims 2 to 4, characterized in that said voltage controlled oscillating circuit means (39) includes ramp circuit means (46, 57, 58) for generating a ramp voltage waveform and

that said regulating circuit means (102) includes first comparison circuit means (60) responsive to said ramp voltage waveform and responsive to said first electrical signal for producing a duty cycle modulation signal when said ramp voltage exceeds the voltage of said first electrical signal and applying said duty cycle modulation signal to said control input (67).

6. Apparatus as claimed in one of the claims 2 to 5, characterized in that said circuit (1) further includes a power source switch that, when closed, supplies operating power to said circuit, that said delay circuit means (106) includes circuit means (95, 96) for producing a relatively slowly rising signal, and comparison circuit means (92) for comparing said relatively slowly rising signal with a predetermined threshold to produce a third electrical signal (86), and

that said regulating circuit means (102) is responsive to said third electrical signal (86) to cause said duty cycle signal to have said predetermined duty cycle.

7. Apparatus as claimed in claim 6, characterized in that said regulating circuit means (102) includes an integrating circuit means (61) for producing an average voltage of said duty cycle signal, a voltage scaling circuit (87-90) responsive to said first electrical signal (38) for producing a fourth electrical signal, and second comparison circuit means (79) responsive to said fourth electrical signal for producing a signal which is conducted to an input of said first comparison means (60) in order to cause said first comparison means (60) to increase or decrease the duty cycle of said duty cycle signal as necessary to cause said average of said duty cycle signal to be equal to the voltage of said fourth electrical signal.

8. Apparatus as claimed in one of the claims 1 to 7, characterized in that said oscillating circuit means (66) includes ring oscillator circuit means (107, 108) for producing first and second substantially non-overlapping signals and first and second field effect transistors (71, 74) responsive, respectively, to said first and second substantially non-overlapping signals and having their drain electrodes coupled, respectively, to the primary winding terminals of a transformer (72), the output terminals (77A, 77B) of said transformer (72) being coupled to said first and second current probes (9A, 9B), respectively.

9. Apparatus as claimed in one of the claims 1 to 8, characterized in that said first and second probes are adapted to make efficient electrical and thermal contact with the surface of cancerous or tumorous tissue in the eye of a livestock animal.

10. Apparatus as claimed in one of the claims 1 to 9, characterized in that said high frequency signal has a frequency of approximately two megahertz.

11. Apparatus as claimed in one of the claims 1 to 10, characterized in that said regulating circuit means (102) operates to reduce the amount of heating of said tissue by said high frequency current in the temperature range from approximately 50°C to 55°C.

12. Apparatus as claimed in one of the claims 1 to 11, characterized in that said first and second predetermined frequencies are approximately 1000 and 4000 cycles per second, respectively, and said first and second predetermined temperature levels are approximately 50°C and 55°C, respectively.

**Patentansprüche**

1. Gerät zur elektrothermischen Behandlung von krankem Gewebe mit einer Schaltung (1) zum Erzeugen eines Flusses eines hochfrequenten Signales durch das kranke Gewebe,

wobei das elektrothermische Gerät erste und zweite, voneinander beabstandete Stromsonden (9A, 9B) aufweist, die jeweils eine Kontaktfläche zur elektrischen Kontaktierung der Oberfläche des kranken Gewebes haben,

wobei die Schaltung eine Ausgangsoszillationsschaltungseinrichtung (76) zum Erzeugen eines Hochfrequenzsignales für ein Anlegen über die erste und zweite Stromsonde (9A, 9B) aufweist,

wobei die Ausgangsoszillationseinrichtung einen Steuereingang (67) hat,

wobei die Schaltung folgendes aufweist:

(a) eine Temperaturfühlereinrichtung (17) zum Erfassen der Temperatur der ersten Stromsonde zum Erzeugen eines ersten elektrischen Signales (38), das die Temperatur der ersten Sonde darstellt;

dadurch gekennzeichnet, daß die Schaltung ferner folgendes aufweist:

(b) eine Verzögerungsschaltungseinrichtung (106) zum Vermindern des Lastzyklus des Hochfrequenzsignales auf einen vorbestimmten Pegel während einer vorbestimmten anfänglichen Zeitdauer nach dem anfänglichen Anlegen der Betriebsleistung an die Schaltung;

(c) eine Einrichtung (60, 79), die den Steuereingang (67) ermittelt, um die Oszillation der Ausgangsoszillationsschaltungseinrichtung (76) anzuhalten, wenn der Steuereingang (67) sich bei einem ersten Signalpegel befindet; und

(d) eine Regelschaltungseinrichtung (102), die auf das erste elektrische Signal (38) anspricht und mit dem Steuereingang (67) verbunden ist, um periodisch das Oszillieren der Ausgangsoszillationsschaltungseinrichtung nach einer vorbestimmten anfänglichen Zeitdauer zu unterbrechen, um ein Lastverhältnis des Hochfrequenzsignales zu vermindern.

2. Gerät nach Anspruch 1, dadurch gekennzeichnet, daß die Ausgangsoszillationsschaltungseinrichtung folgendes aufweist:

(e) eine spannungsgesteuerte Oszillationsschaltungseinrichtung (39), die auf das erste elektrische Signal (38) anspricht, um ein zweites elektrisches Signal (36) zu erzeugen, und um die Frequenz des zweiten elektrischen Signales gemäß Änderungen der Temperatur der ersten Stromsonde (9A) zu variieren, wobei das zweite elektrische Signal eine Frequenz hat, die sich zwischen einer ersten und einer zweiten vorbestimmten Audiofrequenz ändert, wenn sich die Temperatur der ersten Stromsonde zwischen einem ersten und einem zweiten vorbestimmten Temperaturpegel ändert; und

daß die Schaltung (1) ferner aufweist:

(f) eine Audioübertragereinrichtung (34), die auf das zweite elektrische Signal anspricht, um einen hörbaren Klang zu erzeugen, dessen Tonhöhe die Temperatur des Gewebes darstellt, wenn das Gewebe durch das Hochfrequenzsignal erwärmt wird.

3. Gerät nach Anspruch 1 oder 2, dadurch gekennzeichnet, daß die Regelschaltungseinrichtung (102) auf die Verzögerungsschaltungseinrichtung (106) anspricht, um den Lastzyklus des Lastzyklussignales auf den vorbestimmten Lastzyklus während der vorbestimmten Zeitdauer unabhängig von der Temperatur der ersten Stromsonde (9A) zu bringen.

4. Gerät nach einem der Ansprüche 1 bis 3, gekennzeichnet durch eine Schwellenschaltungseinrichtung (26), die auf das erste elektrische Signal anspricht, um ein drittes elektrisches Signal zu erzeugen, wenn das erste elektrische Signal einen vorbestimmten Pegel überschreitet, und eine Zeitgabeschaltungseinrichtung (32), die auf das dritte elektrische Signal anspricht und an die Audioübertragereinrichtung angeschlossen ist, um eine Unterbrechung des Audioklanges mit einer vorbestimmten Wiederholungsrate zu bewirken.

5. Gerät nach einem der Ansprüche 2 bis 4, dadurch gekennzeichnet, daß die spannungsgesteuerte Oszillationsschaltungseinrichtung (39) eine Rampenschaltungseinrichtung (46, 57, 58) zum Erzeugen eines rampenförmigen Spannungssignales aufweist, und daß die Regelschaltungseinrichtung (102) eine erste Vergleichsschaltungseinrichtung (60) aufweist, die auf das rampenförmige Spannungssignal anspricht, und die auf das erste elektrische Signal anspricht, um ein Lastzyklusmodulationssignal zu erzeugen, wenn das rampenförmige Spannungssignal die Spannung des ersten elektrischen Signals überschreitet, und um das Lastzyklusmodulationssignal an den Steuereingang (67) anzulegen.

6. Gerät nach einem der Ansprüche 2 bis 5, dadurch gekennzeichnet, daß die Schaltung ferner einen Leistungsquellenschalter aufweist, der in seinem geschlossenen Zustand Betriebsleistung zu der Schaltung zuführt, daß die Verzögerungsschaltungseinrichtung (106) eine Schaltungseinrichtung (95, 96) zum Erzeugen eines relativ langsam ansteigenden Signales und eine Vergleichsschaltungseinrichtung (92) zum Vergleichen des relativ langsam ansteigenden Signales mit einer vorbestimmten Schwelle zum Erzeugen eines dritten elektrischen Signales (86) aufweist, und daß die Regelschaltungseinrichtung (102) auf das dritte elektrische Signal (86) anspricht, um das Lastzyklussignal auf den vorbestimmten Lastzyklus zu bringen.

7. Gerät nach Anspruch 6, dadurch gekennzeichnet, daß die Regelschaltungseinrichtung (102) eine integrierende Schaltungseinrichtung (61) zum Erzeugen einer mittleren Spannung des Lastzyklussignales, eine Spannungsskalierungsschaltung (67 bis 90), die auf das erste elektrische Signal (38) zum Erzeugen eines vierten elektrischen Signales anspricht, und eine zweite Vergleichsschaltungseinrichtung (79) aufweist, die auf das vierte elektrische Signal anspricht, um ein Signal zu erzeugen, das zu einem Eingang der ersten Vergleichseinrichtung (60) geleitet wird, um die erste Vergleichseinrichtung (60) zum Erhöhen oder Vermindern des Lastzyklussignales zu veranlassen, wie dies nötig ist, um den Mittelwert des Lastzyklussignales an die Spannung des vierten elektrischen Signales anzugleichen.

8. Gerät nach einem der Ansprüche 1 bis 7, dadurch gekennzeichnet, daß die Oszillationsschaltungseinrichtung (66) eine Ringoszillatorschaltungseinrichtung (107, 108) zum Erzeugen eines ersten und zweiten, im wesentlichen nicht überlappenden Signales sowie einen ersten und einen zweiten Feldeffekttransistor (71, 74) aufweist, die jeweils auf das erste und zweite im wesentlichen nicht überlappende Signal ansprechen und mit ihren Drainelektroden jeweils an die Primärwicklungsanschlüsse eines Transformators (72) angeschlossen sind, wobei die Ausgangsanschlüsse (77a, 77b) des Transformators

9

(72) jeweils an die erste und zweite Stromsonde (9A, 9B) angeschlossen sind.

9. Gerät nach einem der Ansprüche 1 bis 8, dadurch gekennzeichnet, daß die erste und zweite Sonde für einen wirkungsvollen elektrischen und thermischen Kontakt mit der Oberfläche des krebsartigen oder tumorartigen Gewebes in dem Auge eines Tieres aus einer Viehzucht geeignet sind.

10. Gerät nach einem der Ansprüche 1 bis 9, dadurch gekennzeichnet, daß das Hochfrequenzsignal eine Frequenz von ungefähr zwei Megahertz hat.

11. Gerät nach einem der Ansprüche 1 bis 10, dadurch gekennzeichnet, daß die Regelschaltungseinrichtung (102) arbeitet, um die Wärmemenge des Gewebes durch den Hochfrequenzstrom in dem Temperaturbereich zwischen ungefähr 50°C und 55°C zu vermindern.

12. Gerät nach einem der Ansprüche 1 bis 11, dadurch gekennzeichnet, daß die erste und zweite vorbestimmte Frequenz bei 1000 und 4000 Zyklen pro Sekunde liegen, und daß die ersten und zweiten vorbestimmten Temperaturpegel bei 50°C und 55°C liegen.

**Revendications**

1. Appareil pour le traitement par électrothermie d'un tissu malade, comprenant un circuit (1) destiné à engendrer la propagation d'un signal à haute fréquence à travers le tissu malade, ledit appareil d'électrothermie comprenant des première et seconde électrodes de courant (9A, 9B) distantes l'une de l'autre et présentant chacune une surface de contact destinée à venir en contact électrique avec la surface dudit tissu malade, ledit circuit comprenant des moyens (66) formant un circuit oscillant de sortie pour générer un signal à haute fréquence destiné à être appliqué entre lesdites première et seconde électrodes de courant (9A, 9B), lesdits moyens formant circuit oscillant de sortie comportant une entrée de commande (67), ledit circuit comprenant:

a) des moyens de détection de température (17) pour détecter la température de ladite première électrode de courant afin de générer un premier signal électrique (38) représentatif de la température de ladite première électrode;

caractérisé en ce que ledit circuit comprend en outre:

b) des moyens formant circuit de temporisation (106) pour réduire la durée utile dudit signal à haute fréquence à un niveau prédéterminé sur une période de temps initiale prédéterminée à la suite de l'application initiale de l'énergie de fonctionnement audit circuit;

c) des moyens (60, 79) déterminant ladite entrée de commande (67) pour stopper l'oscillation desdits moyens (66) formant circuit oscillant de sortie, lorsque ladite entrée de commande (67) est à un premier niveau de signal; et

d) des moyens formant circuit de régulation (102) qui sont sensibles audit premier signal électrique (38) et sont reliés à ladite entrée de commande (67) pour interrompre périodiquement l'oscillation desdits moyens formant circuit oscillant de sortie, au-delà de ladite période de temps initiale prédéterminée afin de réduire une durée utile dudit signal à haute fréquence.

2. Appareil tel que défini dans la revendication 1, caractérisé en ce que lesdits moyens formant circuit oscillant de sortie comprennent:

e) un moyen formant circuit oscillant contrôlé en tension (39) sensible audit premier signal électrique (38) pour générer un second signal électrique (36) et faire varier la fréquence dudit second signal électrique en fonction des variations de température de ladite première électrode de courant (9A), ledit second signal électrique présentant une fréquence qui varie entre une première et une seconde fréquences acoustiques prédéterminées lorsque la température de ladite première électrode de courant varie entre un premier et un second niveaux prédéterminés de température; et

en ce que ledit circuit (1) comprend en outre:

f) des moyens formant transducteur acoustique (34) sensibles audit second signal électrique pour produire un signal sonore audible dont la fréquence représente la température dudit tissu pendant que celui-ci est chauffé par ledit signal à haute fréquence.

3. Appareil tel que défini dans la revendication 1 ou 2, caractérisé en ce que lesdits moyens formant circuit de régulation (102) sont sensibles auxdits moyens formant circuit de temporisation (106) pour amener la durée utile dudit signal de durée utile à prendre ladite valeur prédéterminée de durée utile au cours de ladite période de temps prédéterminée quelle que soit la température de ladite première électrode de courant (9A).

4. Appareil tel que défini dans l'une des revendications 1 à 3, caractérisé par des moyens formant circuit à seuil (26) sensibles audit premier signal électrique pour générer un troisième signal électrique si ledit premier signal électrique dépasse un niveau prédéterminé et

des moyens formant circuit cadenceur (32) sensibles audit troisième signal électrique et reliés auxdits moyens formant transducteur acoustique pour réaliser l'interruption dudit signal sonore à une vitesse de répétition prédéterminée.

5. Appareil tel que défini dans l'une des revendications 2 à 4, caractérisé en ce que ledit moyen formant circuit oscillant contrôlé en tension (39) comprend des moyens (46, 57, 58) formant un circuit générateur d'un signal de tension en dents de scie, et

en ce que lesdits moyens formant circuit de régulation (102) comprennent un premier moyen formant circuit de comparaison (60) sensible audit signal de tension en dents de scie et sensible audit premier signal électrique pour générer un signal de modulation de la durée utile lorsque ladite tension en dents de scie dépasse la tension dudit premier signal électrique et pour appliquer ledit signal de modulation de durée utile sur ladite entrée de commande (67).

6. Appareil tel que défini dans l'une des revendications 2 à 5, caractérisé en ce que ledit circuit (1) comprend en outre un interrupteur d'alimentation électrique qui, lorsqu'il est fermé, assure l'alimentation en énergie de fonctionnement dudit circuit, en ce que lesdits moyens formant circuit de temporisation (106) comprennent un moyen (95, 96) formant un circuit de génération d'un signal à croissance relativement lente, et un moyen formant circuit de comparaison (92) pour comparer ledit signal à croissance relativement lente à un seuil prédéterminé, afin de générer un troisième signal électrique (86), et

en ce que lesdits moyens formant circuit de régulation (102) sont sensibles audit troisième signal électrique (86) pour amener ledit signal de durée utile à prendre ladite valeur prédéterminée de durée utile.

7. Appareil tel que défini dans la revendication 6, caractérisé en ce que lesdits moyens formant circuit de régulation (102) comprennent un moyen formant circuit intégrateur (61) destiné à générer une tension moyenne dudit signal de durée utile, un circuit d'étalonnage de tension (87-90) sensible audit premier signal électrique (38) pour générer un quatrième signal électrique, et un second moyen formant circuit de comparaison (79) sensible audit quatrième signal électrique pour générer un signal qui est amené à une entrée dudit premier moyen de comparaison (60) afin que ledit premier moyen de comparaison (60) provoque une augmentation ou une diminution de la durée utile dudit signal de durée utile si cela est nécessaire pour rendre ladite moyenne dudit signal de durée utile égale à la tension dudit quatrième signal électrique.

8. Appareil tel que défini dans l'une des revendications 1 à 7, caractérisé en ce que lesdits moyens formant circuit oscillant (66) comprennent un moyen formant circuit d'oscillateur en anneau (107, 108) pour générer des premier et second signaux sensiblement non chevauchant, ainsi que des premier et second transistors à effet de champ (71, 74) sensibles respectivement auxdits premier et second signaux sensiblement non chevauchant et ayant leurs drains reliés respectivement aux bornes de l'enroulement primaire d'un transformateur (72), les bornes de sortie (77A, 77B) dudit transformateur (72) étant reliées respectivement auxdites première et seconde électrodes de courant (9A, 9B).

9. Appareil tel que défini dans l'une des revendications 1 à 8, caractérisé en ce que lesdites première et seconde électrodes sont conçues pour réaliser un contact électrique et thermique efficace avec la surface d'un tissu cancéreux ou tumoral de l'oeil d'un animal de bétail.

10. Appareil tel que défini dans l'une des revendications 1 à 9, caractérisé en ce ledit signal à haute fréquence présente une fréquence d'environ deux mégahertz.

11. Appareil tel que défini dans l'une des revendications 1 à 10, caractérisé en ce que lesdits moyens formant circuit de régulation (102) agissent pour réduire le degré de chauffage dudit tissu par ledit courant à haute fréquence dans une plage de températures d'environ 50°C à 55°C.

12. Appareil tel que défini dans l'une des revendications 1 à 11, caractérisé en ce que lesdites première et seconde fréquences prédéterminées sont respectivement d'environ 1000 et 4000 cycles par seconde, et lesdits premier et second niveaux de température prédéterminés sont respectivement d'environ 50°C et 55°C.

Fig-1

Fig-2

**FIG. 3**

**FIG. 3A**

**FIG. 4**